(19) **Europäisches Patentamt**
**European Patent Office**
**Office européen des brevets**

(11) **EP 4 621 434 A1**

(12) **EUROPEAN PATENT APPLICATION**

(43) Date of publication:
24.09.2025 Bulletin 2025/39

(21) Application number: 24164455.8

(22) Date of filing: 19.03.2024

(51) International Patent Classification (IPC):
*G01R 33/563* (2006.01)   *G01R 33/565* (2006.01)

(52) Cooperative Patent Classification (CPC):
**G01R 33/56358; G01R 33/56581**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Siemens Healthineers AG**
  **91301 Forchheim (DE)**
- **Centre National de la Recherche Scientifique (CNRS)**
  **75016 Paris Cedex 16 (FR)**
- **INSTITUT NATIONAL DE LA SANTE ET DE LA RECHERCHE MEDICALE - INSERM**
  **75013 Paris (FR)**

- **King's College London**
  **London WC2R 2LS (GB)**
- **Université Paris XIII Paris-Nord**
  **93430 Villetaneuse (FR)**
- **Université Paris Cité**
  **75006 Paris (FR)**

(72) Inventors:
- **DARWISH, Omar**
  **SE8 5EP London (GB)**
- **NEJI, Radhouene**
  **N65XP London (GB)**
- **SINKUS, Ralph**
  **95620 Parmain (FR)**

(74) Representative: **Siemens Healthineers Patent Attorneys**
**Postfach 22 16 34**
**80506 München (DE)**

(54) **CHARACTERIZATION OF CONCOMITANT FIELD EFFECTS ON MR IMAGING**

(57) The present invention relates to a method of performing 3D Magnetic Resonance Imaging including the step of applying a magnetic gradient field that causes a concomitant field $B_c$. A further step of the method includes determining phase accruals due to the self-squared terms of the concomitant field $B_c$ and phase accruals $\varphi_{xz}$, $\varphi_{yz}$, due to the cross terms of the concomitant field $B_c$ based on an encoding matrix that accounts for the different possible sign combinations of the applied magnetic gradients.

FIG 3

**Description**

The present invention relates to a method of performing 3D Magnetic Resonance Imaging (3D MRI, preferably 3D Magnetic Resonance Elastography (3D MRE) including the steps of applying a magnetic gradient field and determining phase accruals. Furthermore, the present invention relates to a respective 3D magnetic resonance imaging system and a computer program as well as a computer-readable medium.

Independent of the grammatical term usage, individuals with male, female or other gender identities are included within the term.

Hepatic three-dimensional MR Elastography (3D MRE) has shown promises for measuring liver fibrosis and grading liver inflammation using viscoelastic parameters derived from the 3D displacement field as discussed in Shi Y, Qi YF, Lan GY, et al. Three-dimensional MR Elastography Depicts Liver Inflammation, Fibrosis, and Portal Hypertension in Chronic Hepatitis B or C. Radiology. Oct 2021;301(1):154-162, doi:10.1148/radiol.2021202804 and in Darwish OI, Gharib AM, Jeljeli S, et al. Single Breath-Hold 3-Dimensional Magnetic Resonance Elastography Depicts Liver Fibrosis and Inflammation in Obese Patients. Invest Radiol, Jun 1 2023;58(6):413-419, doi:10.1097/rli.0000000000000952.

Fibrosis is measured using the shear wave speed (Cs [m/s]) and inflammation is graded with the loss modulus (G" [kPa]) (compare Sinkus R, Lambert S, Abd-Elmoniem KZ, et al. Rheological determinants for simultaneous staging of hepatic fibrosis and inflammation in patients with chronic liver disease. NMR Biomed . Oct 2018;31(10):e3956, doi :10.1002/nbm.3956).

Hepatic 3D MRE has been limited to high field MR systems ($B0 \geq 1.5T$). Nonetheless, expanding hepatic 3D MRE to wide-bore low field MR systems ($B0 \leq 1.0T$) can serve as a means of accommodating liver patients with high BMI and/or claustrophobia such as non-alcoholic fatty liver disease (NAFLD) patients. NAFLD is a growing global healthy crisis and enabling access to 3D MRE for NAFLD patients is becoming of importance in the clinic. Furthermore, the longer T2* relaxation times at low field may be beneficial to mitigate iron overload in NAFLD patients. Another aspect to consider is that low field MR system bring down the financial entry point of MR which might allow a wider spread of hepatic 3D MRE in middle-income countries.

However, low field MR systems come with a penalty in signal-to-noise ratio (SNR) which is directly proportional to a penalty in phase-to-noise ratio (PNR) in 3D MRE. A Hadamard motion encoding scheme may be used to increase sensitivity to motion, thereby mitigating the decrease in PNR (compare Guenthner C, Runge JH, Sinkus R, Kozerke S. Analysis and improvement of motion encoding in magnetic resonance elastography. NMR Biomed . May 2018;31(5):e3908. doi:10.1002/nbm.3908).

Hadamard motion encoding applies unique combinations of motion encoding gradients on all the gradient axes simultaneously; this however leads to concomitant fields which are higher with decreasing static magnetic field B0 (Bernstein MA, Zhou XJ, Polzin JA, et al. Concomitant gradient terms in phase contrast MR: Analysis and correction. Magnetic Resonance in Medicine, 1998;39(2):300-308, doi: https://doi.org/10.1002/mrm.1910390218).

The object of the present invention is to provide a 3D MRI method and device with improved phase-to-noise ratio.

According to the present invention, this object is solved by a method and a system according to the independent claims. Furthermore, a respective computer program and a computer-readable medium are provided. Favorable further developments of the method and the system are defined in the subclaims.

Accordingly, there is provided a method of performing 3D Magnetic Resonance Imaging including the steps of

-   applying a magnetic gradient field that causes a concomitant field $B_c$ leading to a phase accrual

$$\varphi_c = \frac{\gamma T}{2B_0}\left( \overbrace{G_x^2 z^2 + G_y^2 z^2 + G_z^2 \frac{x^2+y^2}{4}}^{\text{"self-squared" terms}} \underbrace{-\ G_x\,G_z\,x\,z\ -\ G_y\,G_z\,y\,z}_{\text{"cross" terms}} \right)$$

the terms including $G_x^2$, $G_y^2$ and $G_z^2$ are called self-squared terms and the terms including $G_xG_z$ and $G_yG_z$ are called cross terms, wherein x, y and z are coordinates of a 3D space, $B_0$ is a static magnetic field, $G_x$, $G_y$ and $G_z$ are (interleaved) applied magnetic gradients, $\gamma$ is the gyromagnetic ratio characteristic of the nuclei and T is the total time duration for applying the magnetic gradients, and

-   determining phase accruals due to the self-squared terms of the concomitant field $B_c$ and phase accruals $\varphi_{xz}$, $\varphi_{yz}$ due to the cross terms of the concomitant field $B_c$ based on an encoding matrix that accounts for the different possible sign combinations of the applied magnetic gradients.

Whenever a desired linear magnetic field gradient is activated, additional magnetic fields with nonlinear spacial dependence result (compare Bernstein et al.). This is a consequence of Maxwell's Equations for the divergence and curl of the magnetic field. The above-formulated concomitant field $B_c$ is the consequence. The concomitant field affects the phase contrast. Specifically, the phase accumulated by transverse magnetization subjected to the concomitant field is

$$\varphi_c(x, y, z) = \gamma \int B_c(x, y, z, t)\, dt$$

wherein $\gamma$ is the gyromagnetic ratio characteristic of the nuclei.

Advantageously such a 3D MRI/MRE method is easily implemented. Furthermore, MRI/MRE measurements are possible on all fields strenghts. There is no limitation to 0,55 T or Hadamard motion encoding. Specifically, there can be provided a rapid 3D slap-selective MR elastography using interleaved motion encoding.

In an advantageous embodiment MRE phase measurements for determining the phase accruals are performed according to an encoding scheme represented by a pregiven invertible encoding matrix. The encoding matrix is invertible. This means that the equation system can be solved unambiguously.

According to a further embodiment at least or exactly five (MRI or MRE) phase measurements are performed for unambiguously determining the phase accruals due to the self-squared terms of the concomitant field $B_c$ and due to the crossterms of the concomitant field $B_c$, and wherein the encoding matrix is a $5 \times 5$ matrix. Thus, there is an equation system including five unknowns. Each equation includes a phase measurement result and one phase accrual out of the self-squared terms and the crossterms. If an adequate 5x5 encoding matrix is used, the phase accruals can be determined unambiguously.

In an alternative embodiment at least or exactly six (e.g. MRE) phase measurements $m_1$ to $m_6$ are performed for clearly determining the phase accruals $\varphi_{Uz}$, $\varphi_{Ux}$, $\varphi_{Uy}$ due to a 3D displacement field and the phase accruals $\varphi_{xz}$, $\varphi_{yz}$ due to the cross terms of the concomitant field $B_c$ as well as a phase accrual $\varphi_{err}$ due to a constant phase error and due to the self squared terms of the concomitant field Bc, and wherein the encoding matrix M is a 6x6 matrix, wherein

$$\begin{bmatrix} m_1 \\ m_2 \\ m_3 \\ m_4 \\ m_5 \\ m_6 \end{bmatrix} = M \begin{bmatrix} \varphi_{U_z} \\ \varphi_{U_x} \\ \varphi_{U_y} \\ \varphi_{err} \\ \varphi_{xz} \\ \varphi_{yz} \end{bmatrix}$$

Beside the phase accruals due to the crossterms typically a phase accrual due to a constant phase error and due to the self-squared terms occurs. If this phase error is unknown, it can be integrated into the equation system including six equations and six unknowns. Thus, no more than six phase measurements are necessary to determine the six phase accruals. If a further component of the phase accrual has to be determined, beside the six unknowns, even a 7x7 encoding matrix may be used. In this case at least seven phase measurements are necessary to unambiguously determine the seven unknowns. In other words, at least five phase measurements are necessary to determine the phase accruals due to the self-squared terms and the crossterms of the concomitant field $B_c$. If more parameters have to be determined, more than five phase measurements have to be performed and higher squared matrixes than 5x5 must be used.

According to a further embodiment, the invertible encoding matrix only includes elements like -1 and +1. This means that motion encoding gradients applied on all gradient axes are bipolar. For instance, the bipolar motion encoding gradient G may include two lopes $G_1$ and $G_2$, wherein $G_2 = -G_1$.

In another embodiment the invertible encoding matrix includes elements that reflect the amplitudes of the magnetic gradients applied. This means that the amplitudes do not always have to be the same among each other.

In a specific embodiment the $6 \times 6$ matrix is equal to

$$M = \begin{bmatrix} -1 & +1 & -1 & +1 & +1 & -1 \\ +1 & -1 & -1 & +1 & +1 & +1 \\ -1 & -1 & +1 & +1 & -1 & +1 \\ +1 & +1 & +1 & +1 & -1 & -1 \\ +1 & -1 & +1 & +1 & +1 & -1 \\ -1 & +1 & +1 & +1 & +1 & +1 \end{bmatrix}$$

or any other matrix having the same rows like M.

This $6\times6$ encoding matrix is a further development of the $4\times4$ hadamard matrix. The 6x6 encoding matrix allows for unambiguously solving the self-squared terms, the crossterms and the phase error. Thus, the respective encoded MRI or MRE measurement consists of unique combinations of motion encoding gradients applied on all gradient axes simultaneously with equal amplitudes. Specifically, the 6x6 encoding matrix accounts for the different possible sign combinations of the crossterms. The same results can be obtained by using matrices having the same rows like M but in a different order.

According to a preferred embodiment, the magnetic gradients $G_x$, $G_y$ and $G_z$ have equal amplitudes. For instance, the gradients of the above 6x6 matrix are equal in amplitude and have different signs. However, similar results may be obtained by using gradient amplitudes different from each other. Furthermore, two of the gradients may have the same amplitude whereas the third gradient has a different amplitude. Such encoding scheme may be used in order to emphasize one spacial direction.

According to a further embodiment, the motion encoding gradients are applied without any overlap with the imaging gradients. Thus, the motion encoding gradients are temporarily separated from the imaging gradients, thereby obtaining unambiguous stimulations.

In another embodiment a 2D or 3D image is displayed based on one of the phase accruals $\varphi_{Uz}$, $\varphi_{Ux}$, $\varphi_{Uy}$, $\varphi_{xz}$, $\varphi_{yz}$. Such images provide information about the elasticity and viscosity of an object under investigation.

According to a further embodiment, a 2D or 3D image is displayed based on an amplitude of a temporal Fourier transform of the phase accruals $\varphi_{Uz}$, $\varphi_{Ux}$, $\varphi_{Uy}$, $\varphi_{xz}$, $\varphi_{yz}$. The Fourier transforms show the spectral distribution of the phase accruals at a specific mechanical vibration frequency (e.g. 60Hz).

In a preferred embodiment any of the above methods is performed as part of a 3D Magnetic Resonance Elastography (MRE). Thus, concomitant field effects can be quantified and assessed in MR Elastography. However, the above methods can be used for other MR techniques too, where additional gradients are applied besides the imaging gradients.

In a specific embodiment the mechanical excitation is performed at a frequency in the range of 20 to 100 Hz, preferably at 60 Hz. Such frequencies enable high quality in MRE measurements of human tissue.

The above object is also solved by a 3D Magnetic Resonance Imaging (MRI) system including a magnet device for applying magnet gradient field and an evaluation device for determining phase accruals, wherein the system is configured to perform a method as described above. Specifically, there may be provided a 3D MRE system including the above magnet device and evaluation device as well as an actuator for generating sheer waves.

Moreover, there is provided a computer program comprising instructions which, when the program is executed by a 3D magnetic resonance imaging system, cause the magnetic resonance imaging system to carry out the method described above. Furthermore, there may be provided a computer-readable medium comprising instructions, which, when executed by a 3D magnetic resonance imaging system, cause the magnetic resonance imaging system to carry out the method described above.

The present invention will now be described in more detail in connection with the attached drawings showing in:

FIG 1 a schematical diagram of an embodiment of a MRE system;

FIG 2 unwrapped phases and Fourier transforms of phantom measurements without mechanical excitation and

FIG 3 unwrapped phases and Fourier transforms of the phantom measurement with mechanical excitation.

The following embodiments represent preferable examples of the present invention.

The following embodiments relate to MRE systems and methods. However, they can be used for other MR techniques too where additional gradients are applied besides the imaging gradients.

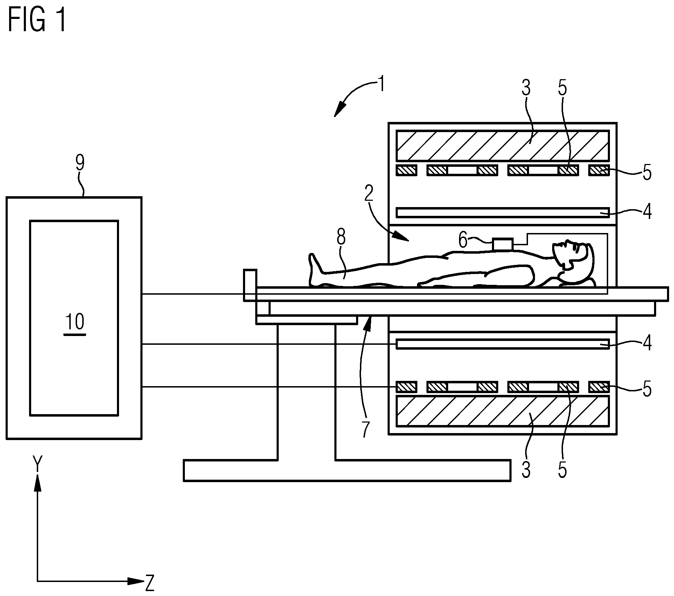

FIG 1 shows a schematic representation of an exemplary implementation of an MRE-system 1 according to the invention.

The MRE-system comprises a magnet unit with an imaging region 2, for example within a patient tunnel for placing an object 8, in particular a patient, to be imaged. The magnet unit comprises a field magnet 3 that generates a main magnet field for aligning nuclear spins of the object, in particular within the imaging region 2. The imaging region 2 is characterized by a very homogeneous static main magnet field, the homogeneity relating, in particular, to the magnetic field strength. The field magnet 3 may, for example, be a superconducting magnet capable of providing magnetic fields with a magnetic flux density in the order of several Tesla, in particular in the order of 7 T or more. A patient table 7 may be movable within the patient tunnel.

Furthermore, the magnet unit comprises a gradient coil arrangement 5 with several gradient coils that are designed to superimpose location-dependent magnetic fields in the three spatial directions on the static main magnet field for spatial differentiation and, in particular, slice selection. The gradient coils of the gradient coil arrangement 5 may, for example, be designed as coils of normal conducting wires, which may, for example, generate mutually orthogonal fields or field gradients in the recording region.

The MRE-system 1, in particular the magnet unit, comprises a transmission coil arrangement, which contains one or more RF-coils 4. It is noted that the one or more RF-coils 4 of the transmission coil arrangement may, depending on the specific implementation or application, also be used as receiving coils. Optionally, the MRI-system 1 may also comprise one or more local coils (not shown in FIG 1), which may be arranged in the immediate vicinity of the object 8, for example on the object 8 or in the patient table 7. The local coils may serve as receiving coils and/or transmission coils.

Furthermore, the MRE-system comprises a (pulse) wave generator 6 for exciting shear waves within the object/patient to be examined.

The MRE-system 1 also comprises a data processing apparatus 9 including at least one computing unit 10. The at least one computing unit 10 is configured to carry out a computer-implemented method for controlling the MRE-system according to a specific timing scheme. Particularly, the time control of the wave generator 6 is synchronized with the MR imaging. As a result of the computer-implemented method, the at least one computing unit 10 controlls the of the MRE-system so that the acquisition is performed in a very efficient way.

In response to the excitation RF-pulses, the at least one computing unit 10 receives corresponding NMR-signals from the receiving coils and may generate respective MR-images of the object 8 depending on those signals.

In particular, the at least one computing unit may comprise a readout control unit, which is connected to the at least one RF-coil 4 and/or the local coil. Depending on the detected MR-signals, the readout control unit, which may comprise an analogue-to-digital converter, ADC, may generate corresponding MR-data, in particular in k-space.

The at least one computing unit 10 may evaluate the MR-data and, for example, carry out a two-dimensional or three-dimensional image reconstruction based on the MR-data. The at least one computing unit 10 also comprises a sending control unit, which is connected to and controls the RF-coil(s) 4 and/or the local coil to emit the excitation RF-pulses and, for example refocusing RF-pulses and other RF-pulses. The at least one computing unit 10 comprises gradient control unit, which is connected to and controls the gradient coil arrangement 5 to apply, for example, slice selecting gradients, gradients for frequency and/or phase encoding, defocusing gradients and/or readout gradients and so forth. Additionally, the at least one computing unit 10 comprises a wave generation control unit adapted to control the wave generator.

It is noted that the described structure of the MRI-system 1 is a non-limiting example only. The different required tasks and functions may also be distributed differently and/or to different units in other applications.

Concomitant field effects in MRE might become of importance when translating MRE to low field MR systems. To design an experiment that measures the effect of concomitant fields $B_c$ on Hadamard-encoded 3D MRE, we first look at the phase accrual $\varphi_c$ of $B_c$ following the work of Bernstein et al.:

$$\varphi_c = \frac{\gamma T}{2B_0}\left(\overbrace{G_x^2 z^2 + G_y^2 z^2 + G_z^2\frac{x^2+y^2}{4}}^{\text{"self-squared" terms}} \underbrace{- G_x\,G_z\,xz - G_y\,G_z\,yz}_{\text{"cross" terms}}\right) \quad (1)$$

A Hadamard-encoded MRE measurement consists of unique combinations of motion encoding gradients applied on all gradient axes simultaneously with equal amplitudes. The following 4x4 Hadamard matrix ($H$) is typically used in MRE measurement:

$$H = \begin{bmatrix} -1 & +1 & -1 & +1 \\ +1 & -1 & -1 & +1 \\ -1 & -1 & +1 & +1 \\ +1 & +1 & +1 & +1 \end{bmatrix} \quad (2)$$

The first three terms in equation (1), the "self-squared" terms, are constant throughout the different measurements of Hadamard encoding and are therefore encoded in the same term that encodes constant phase errors such as magnetic field inhomogeneity. However, the last two terms, the "cross" terms, change signs between the different measurements of Hadamard encoding depending on the polarity of the applied motion encoding gradients. As a result, the "cross" terms cannot be resolved using a conventional $4\times4$ Hadamard encoding matrix.

A larger squared encoding matrix (e.g. $5\times5$, $6\times6$, $7\times7$ matrix) can resolve the "cross" terms.

For instance, the Hadamard encoding can be extended to a $6\times6$ encoding matrix M that accounts for the different possible sign combinations of the "cross" terms:

$$M = \begin{bmatrix} -1 & +1 & -1 & +1 & +1 & -1 \\ +1 & -1 & -1 & +1 & +1 & +1 \\ -1 & -1 & +1 & +1 & -1 & +1 \\ +1 & +1 & +1 & +1 & -1 & -1 \\ +1 & -1 & +1 & +1 & +1 & -1 \\ -1 & +1 & +1 & +1 & +1 & +1 \end{bmatrix} \quad (3)$$

$$\begin{bmatrix} m_1 \\ m_2 \\ m_3 \\ m_4 \\ m_5 \\ m_6 \end{bmatrix} = M \begin{bmatrix} \varphi_{U_z} \\ \varphi_{U_x} \\ \varphi_{U_y} \\ \varphi_{err} \\ \widehat{\varphi_{xz}} \\ \widehat{\varphi_{yz}} \end{bmatrix} \quad (4)$$

Where $m_k$ (k=1,2,..,6) is the k-th MRE phase measurement, $\varphi_{Ux}$, $\varphi_{Uy}$, and $\varphi_{Uz}$ are the phase accruals due to the 3D displacement field. $\varphi_{err}$ is the phase accrual due to constant phase errors and the phase accrual of the "self-squared" terms. $\varphi_{xz}$ and $\varphi_{yz}$ are the phase accrual due to the first and second "cross" terms respectively.

The proposed encoding matrix $M$ is incorporated into a previously published 3D MRE sequence (Guenthner et al.) extending the sequence from 4 to 6 measurements. The motion encoding gradients are applied without any overlap with the imaging gradients.

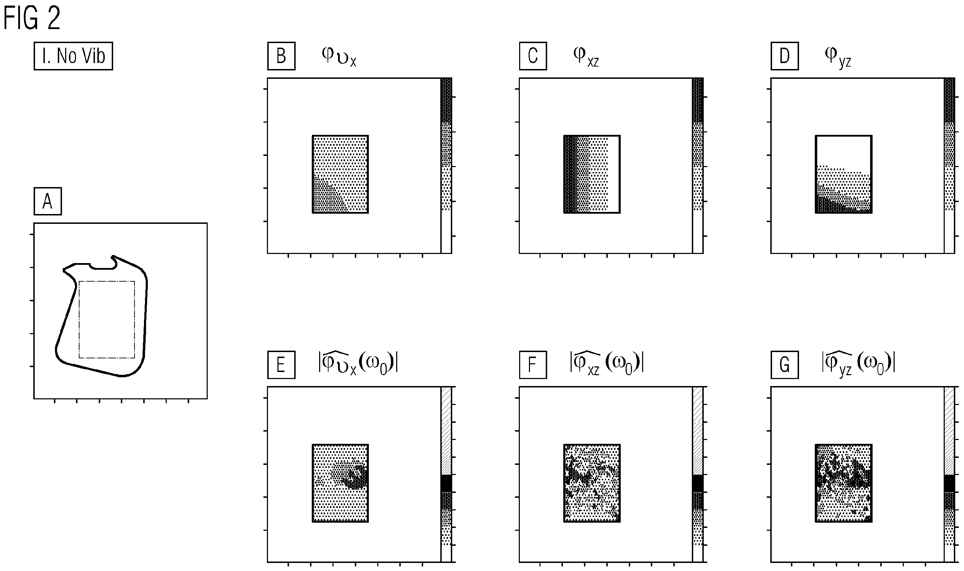

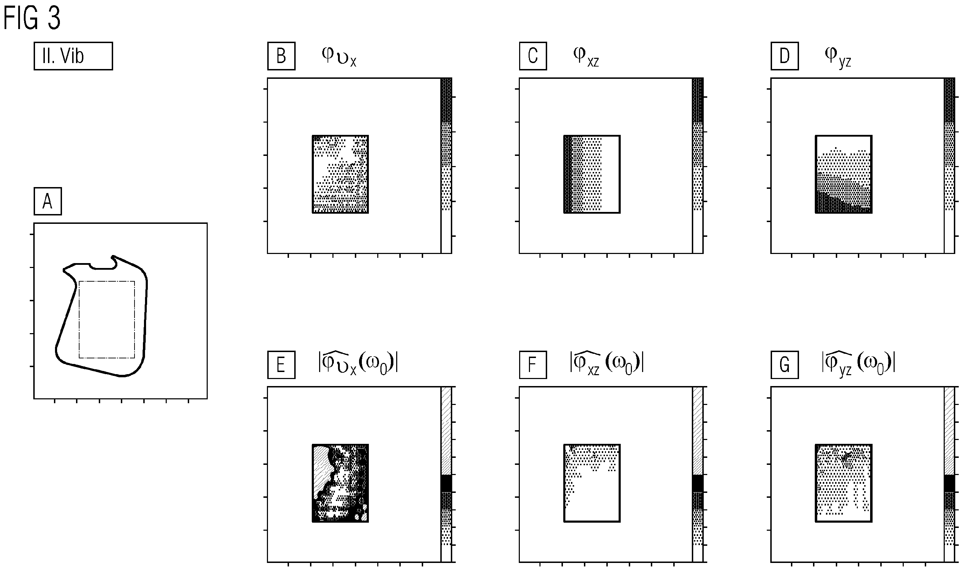

The extended 3D MRE sequence can be implemented on a 0.55T system. Two phantom experiments are conducted: (I) without vibration to verify that $M$ is solving for the "cross" terms (see FIG 2). Note that the first "cross" term ($G_x G_z xz$) varies with $x$ and $z$, and the second "cross" term ($G_y G_z yz$) varies with $y$ and $z$ both independent of the vibration. (II) an experiment with 60Hz mechanical excitation (see FIG 3) to examine the phase accrual due to $B_c$ with respect to the acquired mechanical wave phase offsets. The phantom used is an ultrasound gel phantom shifted to right of the iso-centre of the bore to increase the apparent effect of the concomitant fields.

The results of the phantom experiment without mechanical excitation, i.e. without vibrations, are shown in FIG 2. There is no spatial variation in $\varphi_{Ux}$ (FIG 1.B). However, we can observe a spatial variation in $x$ (left-right direction) in $\varphi_{xz}$ (FIG 2.C) and a spatial variation in $y$ (posterior-anterior direction) in $\varphi_{yz}$ (FIG 2.D). The spatial variation observed in FIG 2.C-D suggests that the proposed encoding matrix $M$ is solving for the "cross" terms. The amplitude of the displacement field in the three terms ($\varphi_{Ux}$, $\varphi_{xz}$, $\varphi_{yz}$) is very low (0.9 $\pm$ 0.2 um, 1.4 $\pm$ 0.2 um, and 1.6 $\pm$ 0.2 um respectively) (FIG 2.E-G).

Specifically, FIG 2.A shows a magnitude image calculated by averaging the central four slices of the acquisiton volume. FIG 2.B-D show the unwrapped phases ($\varphi_{Ux}$, $\varphi_{xz}$, and $\varphi_{yz}$) after decoding of a single slice, and FIG 2.E-G show the amplitude of the temporal Fourier transform ($\widehat{\varphi_{U_x}}$, $\widehat{\varphi_{xz}}$, $\widehat{\varphi_{yz}}$) at the mechanical vibration frequency ($\omega_0$ = 60Hz) of $\varphi_{Ux}$, $\varphi_{xz}$, and $\varphi_{yz}$ respectively averaged over the central four slices.

The results of the phantom experiments with mechanical excitation are presented in FIG 3. $\varphi_{Ux}$ (FIG 3.B) varies with the mechanical excitation, however, $\varphi_{xz}$ (FIG 3.C) and $\varphi_{yz}$ (FIG 3.D) only vary spatially in $x$ (left-right direction) and in $y$ (posterior-anterior direction) respectively. In addition, the amplitude of the displacement field in $\varphi_{Ux}$ (81 $\pm$ 11 um) is approximately an order of magnitude higher than the amplitudes in $\varphi_{xz}$ (5 $\pm$ 2 um) and $\varphi_{yz}$ (10 $\pm$ 2 um) (FIG 3.E-G). Specifically, FIG 3.A shows a magnitude image calculated by averaging the central four slices of the acquisiton volume. FIG 3.B-D show the unwarpped phases ($\varphi_{Ux}$, $\varphi_{xz}$, and $\varphi_{yz}$) after decoding of a single slice, and FIG 3.E-G show the amplitude of the temporal fourier transform ($\widehat{\varphi_{U_x}}$, $\widehat{\varphi_{xz}}$, $\widehat{\varphi_{yz}}$) at the mechanical vibration frequency ($\omega_0$ = 60Hz) of $\varphi_{Ux}$, $\varphi_{xz}$, and $\varphi_{yz}$ respectively averaged over the central four slices.

The inventive method is easy to implement and extend to other MRE measurements on all field strength and is not limited only to 0.55T systems or Hadamard motion encoding.

## Claims

1. Method of performing 3D Magnetic Resonance Imaging including the steps of

- applying a magnetic gradient field that causes a concomitant field $B_c$ leading to a phase accrual

$$\varphi_c = \frac{\gamma T}{2B_0} \left( \overbrace{G_x^2 z^2 + G_y^2 z^2 + G_z^2 \frac{x^2 + y^2}{4}}^{\text{"self-squared" terms}} \underbrace{- G_x\, G_z x\, z - G_y\, G_z y z}_{\text{"cross" terms}} \right)$$

the terms including $G_x^2$, $G_y^2$ and $G_z^2$ are called self-squared terms and the terms including $G_x G_z$ and $G_y G_z$ are called cross terms, wherein x, y and z are coordinates of a 3D space, $B_0$ is a static magnetic field, $G_x$, $G_y$ and $G_z$ are applied magnetic gradients, $\gamma$ is the gyromagnetic ratio characteristic of the nuclei and T is the total time duration for applying the magnetic gradients, and

- determining phase accruals due to the self-squared terms of the concomitant field $B_c$ and phase accruals $\varphi_{xz}$, $\varphi_{yz}$ due to the cross terms of the concomitant field $B_c$ based on an encoding matrix that accounts for the different possible sign combinations of the applied magnetic gradients.

2. Method according to claim 1, wherein phase measurements for determining the phase accruals are performed according to an encoding scheme represented by a pregiven invertible encoding matrix.

3. Method according to claim 2, wherein exactly five phase measurements are performed for unambiously determining the phase accruals due to the self-squared terms of the concomitant field $B_c$ and due to the cross terms of the concomitant field $B_c$, and wherein the encoding matrix is a 5×5 matrix.

4. Method according to claim 2, wherein exactly six phase measurements $m_1$ to $m_6$ are performed for clearly determining phase accruals $\varphi_{Uz}$, $\varphi_{Ux}$, $\varphi_{Uy}$ due to a 3D displacement field and the phase accruals $\varphi_{xz}$, $\varphi_{yz}$ due to the cross terms of the concomitant field $B_c$ as well as a phase accrual $\varphi_{err}$ due to a constant phase error and due to the self squared terms of the concomitant field Bc, and wherein the encoding matrix M is a 6×6 matrix, wherein

$$\begin{bmatrix} m_1 \\ m_2 \\ m_3 \\ m_4 \\ m_5 \\ m_6 \end{bmatrix} = M \begin{bmatrix} \varphi_{Uz} \\ \varphi_{Ux} \\ \varphi_{Uy} \\ \varphi_{err} \\ \varphi_{xz} \\ \varphi_{yz} \end{bmatrix}$$

5. Method according to one of the claims 2 to 4, wherein the invertible encoding matrix only includes elements like -1 and +1.

6. Method according to one of the claims 2 to 4, wherein the invertible encoding matrix includes elements that reflect the amplitudes of the magnetic gradients applied.

7. Method according to claim 4 or 5, wherein the 6×6 matrix is equal to

$$M = \begin{bmatrix} -1 & +1 & -1 & +1 & +1 & -1 \\ +1 & -1 & -1 & +1 & +1 & +1 \\ -1 & -1 & +1 & +1 & -1 & +1 \\ +1 & +1 & +1 & +1 & -1 & -1 \\ +1 & -1 & +1 & +1 & +1 & -1 \\ -1 & +1 & +1 & +1 & +1 & +1 \end{bmatrix}$$

or any other matrix having the same rows like M.

8. Method according to one of the preceding claims, wherein the magnetic gradients $G_x$, $G_y$ and $G_z$ have equal amplitudes.

9. Method according to one of the preceding claims wherein the magnetic gradients Gx Gy Gz have unequal amplitudes

10. Method according to one of the preceding claims, wherein the motion encoding gradients are applied without any overlap with imaging gradients.

11. Method according to one of the claims 4 to 10, including the step of displaying a 2D or 3D image based on one of the phase accruals $\varphi_{Uz}$, $\varphi_{Ux}$, $\varphi_{Uy}$, $\varphi_{xz}$, $\varphi_{yz}$.

12. Method according to one of the claims 4 to 11, including the step of displaying a 2D or 3D image based on an amplitude of a temporal Fourier transform of one of the phase accruals $\varphi_{Uz}$, $\varphi_{Ux}$, $\varphi_{Uy}$, $\varphi_{xz}$, $\varphi_{yz}$.

13. Method according to one of the preceding claims, which is performed as part of a 3D Magnetic Resonance Elastography.

14. Method according to one of the preceding claims, wherein mechanical excitation is performed at a frequency in the range of 20 to 100 Hz, preferably at 60 Hz.

15. 3D Magnetic Resonance Imaging system (1) including a magnet device (3, 4, 5) for applying a magnetic gradient field and an evaluation device (9) for determining phase accruals, wherein the system (1) is configured to perform a method according to one of the preceding claims.

16. A computer program comprising instructions which, when the program is executed by a 3D Magnetic Resonance Imaging system (1) according to claim 15, cause the Magnetic Resonance Imaging system (1) to carry out the method according to one of the claims 1 to 14.

17. A computer-readable medium comprising instructions which, when executed by a 3D Magnetic Resonance Imaging system (1) according to claim 15, cause the Magnetic Resonance Imaging system (1) to carry out the method according to one of the claims 1 to 14.

FIG 1

FIG 2

I. No Vib

A

B    $\varphi_{\upsilon_x}$

C    $\varphi_{xz}$

D    $\varphi_{yz}$

E    $|\widehat{\varphi_{\upsilon_x}}(\omega_0)|$

F    $|\widehat{\varphi_{xz}}(\omega_0)|$

G    $|\widehat{\varphi_{yz}}(\omega_0)|$

EP 4 621 434 A1

FIG 3

Europäisches
Patentamt

European
Patent Office

Office européen
des brevets

# EUROPEAN SEARCH REPORT

Application Number

EP 24 16 4455

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | BERNSTEIN M A ET AL: "CONCOMITANT GRADIENT TERMS IN PHASE CONTRAST MR: ANALYSIS AND CORRECTION", MAGNETIC RESONANCE IN MEDICINE, WILEY-LISS, US, vol. 39, no. 2, 1 February 1998 (1998-02-01), pages 300-308, XP000734742, ISSN: 0740-3194 | 1-6,8-17 | INV. G01R33/563 G01R33/565 |
| A | * the whole document * | 7 | |
| A | US 5 877 629 A (KING KEVIN F [US] ET AL) 2 March 1999 (1999-03-02) * paragraphs [0007], [0008] * | 1-15 | |
| A | US 2016/334490 A1 (TRZASKO JOSHUA D [US] ET AL) 17 November 2016 (2016-11-17) * paragraph [0024]; figure 2 * | 1-15 | |
| A,D | CHRISTIAN GUENTHNER: "Analysis and improvement of motion encoding in magnetic resonance elastography", NMR IN BIOMEDICINE., vol. 31, no. 5, 30 March 2018 (2018-03-30) , XP093156057, GB ISSN: 0952-3480, DOI: 10.1002/nbm.3908 * the whole document * | 1-17 | TECHNICAL FIELDS SEARCHED (IPC) G01R |

The present search report has been drawn up for all claims

| Place of search | Date of completion of the search | Examiner |
|---|---|---|
| Munich | 2 September 2024 | Ó Donnabháin, C |

EPO FORM 1503 03.82 (P04C01)

EP 4 621 434 A1

## ANNEX TO THE EUROPEAN SEARCH REPORT
## ON EUROPEAN PATENT APPLICATION NO.

EP 24 16 4455

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2024

| Patent document cited in search report | | Publication date | Patent family member(s) | Publication date |
|---|---|---|---|---|
| US 5877629 | A | 02-03-1999 | NONE | |
| US 2016334490 | A1 | 17-11-2016 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHI Y** ; **QI YF** ; **LAN GY et al.** Three-dimensional MR Elastography Depicts Liver Inflammation, Fibrosis, and Portal Hypertension in Chronic Hepatitis B or C.. *Radiology*, October 2021, vol. 301 (1), 154-162
- **DARWISH OL** ; **GHARIB AM** ; **JELJELI S et al.** Single Breath-Hold 3-Dimensional Magnetic Resonance Elastography Depicts Liver Fibrosis and Inflammation in Obese Patients. *Invest Radiol*, 01 June 2023, vol. 58 (6), 413-419
- **SINKUS R** ; **LAMBERT S** ; **ABD-ELMONIEM KZ et al.** Rheological determinants for simultaneous staging of hepatic fibrosis and inflammation in patients with chronic liver disease. *NMR Biomed* ., October 2018, vol. 31 (10), e3956
- **GUENTHNER C** ; **RUNGE JH** ; **SINKUS R** ; **KOZERKE S.** Analysis and improvement of motion encoding in magnetic resonance elastography.. *NMR Biomed*, May 2018, vol. 31 (5), e3908
- **BERNSTEIN MA** ; **ZHOU XJ** ; **POLZIN JA et al.** Concomitant gradient terms in phase contrast MR: Analysis and correction.. *Magnetic Resonance in Medicine*, 1998, vol. 39 (2), 300-308